# FASCICULE DE BREVET EUROPEEN

(11) **EP 1 130 010 B1**
(45) Date de publication et mention de la délivrance du brevet: **14.07.2004**
(21) Numéro de dépôt: 01400372.7
(22) Date de dépôt: 13.02.2001
(51) Int. Cl.: C07C 17/10, C07C 17/21, C07C 17/20, C07C 19/12, C07C 19/08

(54) **Procédé de préparation du 1,1,1- trifluoro-2,2-dichloroéthane**
Verfahren zur Herstellung von 1,1,1-Trifluor-2,2-Dichlorethan
Process for the preparation of 1,1,1-trifluoro-2,2-dichloroethane

(30) Priorité: 29.02.2000 FR 0002530
(43) Date de publication de la demande: 05.09.2001
(73) Titulaire: Atofina, 92800 Puteaux (FR)
(72) Inventeur: Jorda, Eric, 69005 Lyon (FR); Lacroix, Eric, 69480 Amberieux d'Azergues (FR); Perdrieux, Sylvain, 69390 Charly (FR)
(74) Mandataire: Pochart, François

(56) Documents cités:
- EP-A- 0 346 612
- EP-A- 0 402 874
- EP-A- 0 462 514
- EP-A- 0 583 703
- EP-A- 0 638 535
- EP-A- 0 687 660
- US-A- 5 723 700

## Description

La présente invention a pour objet un procédé de préparation du 1,1,1-trifluoro-2,2-dichloroéthane (F123) par chloration catalytique du 1,1,1-trifluoro-2-chloroéthane (F133a) en présence de fluorure d'hydrogène (HF). Elle concerne également l'application de ce procédé à un procédé de fabrication du pentafluoroéthane (F125).

Les composés F123 et F125 pouvant être utilisés comme substituts des perchlorofluorocarbures (CFC) dans le domaine des aérosols (agents propulseurs) et dans celui de la réfrigération, on recherche actuellement des procédés performants pour leur production industrielle.

Dans WO 95/16654 on décrit la mise en contact, à une température de 340 °C, du F133a avec du chlore et de l'HF en présence d'un catalyseur au chrome. Bien que la conversion du F133a dans cette réaction soit élevée, elle produit majoritairement, à cette température, du 1,1,1,2-tétrafluoroéthane (F134a). Ainsi, la sélectivité en F123 ne dépasse pas 15 %, ce qui ne permet pas d'envisager une production de ce composé dans des conditions industriellement acceptables.

Dans WO 94/11327 on mentionne la mise en contact, à des températures inférieures à 300 °C, du F133a avec du chlore et de l'HF en présence d'un catalyseur au chrome. Cette réaction est effectuée avec un très fort excès de chlore et d'HF, et conduit préférentiellement à la formation de F124 et de F125 ; ainsi la sélectivité en F123 reste inférieure à 8 % et le ratio série 110/série 120 est supérieur à 10%.

Dans EP-A-526 908 et EP-A-346 612, on propose de préparer du F123 par mise en contact de chlore avec le F133a, à une température se situant de préférence entre 350 et 450 °C, en l'absence ou en présence d'un catalyseur, cette chloration étant effectuée en l'absence d'HF.

Dans US-A-4 145 368, on propose un procédé consistant à faire réagir du chlore avec du F133a, puis à séparer le F123 du milieu réactionnel, à faire réagir le F113a résultant de cette séparation avec une nouvelle quantité de F133a, cette réaction étant conduite en phase vapeur et de préférence entre 350 et 425 °C, en présence d'un catalyseur tel qu'un oxyde de chrome.

Selon ce document, la sélectivité finale en F123 n'excède pas 29 %.

Dans EP-B-407 990 on propose la chloration de F133a en F123 par activation thermique ou catalytique, en phase liquide sous pression. La sélectivité en F123 peut aller de 67,9 à 83,4 %, la pression de réaction allant de 50 à 127 bars.

Dans EP-A-402 874, on propose de faire réagir le chlore avec le F133a entre 350 et 450 °C, en l'absence de catalyseur et de HF. Selon ce document, grâce à une combinaison particulière de conditions de température, de temps de contact et de rapport molaire des réactifs, on peut éliminer la production de F113a.

Dans US-A-5 414 166, on propose une chloration de F133a en présence d'hydrogène, entre 250 et 500 °C et de préférence entre 350 et 450 °C : la sélectivité en F123 peut aller de 65 à 92 %.

Dans US-A-5 723 700, on décrit une étape au cours de laquelle F133a, HF et Cl₂ réagissent, en présence d'un catalyseur de fluoration, entre 300 et 450 °C pour obtenir, essentiellement du F134a et des traces de F123.

EP-A-0 346 612 décrit un procédé de production de 1,1,1-trifluoro-2,2-dichloroéthane (F123) par chloration du 1,1,1-trifluoro-2-chloroéthane (F133a) avec du chlore gazeux en présence ou en l'absence d'un sel métallique en tant que catalyseur.

EP-A-0 402 874 décrit un procédé de production de 1,1,1-trifluoro-2,2-dichloroéthane (F123) par chloration en phase gazeuse du 1,1,1-trifluoro-2-chloroéthane. Le formation du sous-produit 1,1,1-trifluorotrichloroéthane est évitée en mettant en oeuvre la réaction avec un ratio molaire chlore/composés organiques inférieur à 1 et à une température de 350 à 450°C.

US-A-5 723 700 décrit un procédé de production de 1,1,1,2-tetrafluoroéthane et de pentafluoroéthane par réaction de 1,1,1-trifluoro-2-chloroéthane, HF et chlore dans un premier réacteur; le transfert dans un second réacteur avec du trichloroethylene; la séparation du 1,1,1,2-tetrafluoroéthane et du pentafluoroéthane et le recyclage vers le premier réacteur des produits restant.

EP-A-0 583 703 décrit un procédé de préparation du 1,1,1-trifluoro-2-chloroéthane par hydrofluoration en phase gazeuse de trichloroethylene en présence d'un catalyseur à base de Cr₂O₃.

EP-A-0 462 514 décrit un procédé de préparation du 1-chloro-2,2,2-trifluoroéthane par hydrofluoration de trichloroethylene en présence d'un catalyseur à base d'antimoine.

EP-A-0 687 660 décrit un procédé de production de pentafluoroéthane par division de la zone réactionnelle en une première zone pour la réaction en phase gazeuse sous pression élevée de perchloroethylene avec HF et en une seconde zone pour la réaction en phase gazeuse sous pression faible de 1,1,1-trifluoro-2,2-dichloroéthane (F123) et/ou 2-chloro-1,1,1,2-tetrafluoroethane (F124) avec HF.

EP-A-0 638 535 décrit un procédé de préparation de pentafluoroéthane par réaction du 1,1,1-trifluoro-2,2-dichloroéthane (F123) avec HF en présence d'un catalyseur à base de Cr₂O₃.

L'invention propose un procédé de préparation de F123 par chloration de F133a, selon lequel on opère à une température relativement modérée.

L'invention propose également un procédé de préparation de F123 par chloration de F133a, selon lequel on opère à pression atmosphérique ou sous pression modérée, par exemple n'excédant pas 25 bars.

L'invention propose également un procédé de préparation de F123 par chloration de F133a, selon lequel on opère en présence d'un catalyseur de chloration et/ou de fluoration.

L'invention propose également un procédé de préparation de F123 par chloration de F133a, selon lequel on obtient une sélectivité en F123 supérieure à 50 %.

L'invention propose encore un tel procédé, selon lequel la sélectivité en F123 est supérieure à 70 %.

L'invention propose plus précisément un procédé de préparation de 1,1,1-trifluoro-2,2-dichloroéthane (F123) par mise en contact de 1,1,1-trifluoro-2-chloroéthane (F133a) avec du chlore, ledit procédé étant caractérisé en ce que ladite mise en contact est réalisée en présence d'HF et d'un catalyseur dans des conditions de température, de temps de contact et avec des rapports molaires Cl₂/F133a et HF/F133a tels que HF ne réagisse substantiellement pas avec le F133a et le F123 formé et favorise la sélectivité en F123.

Ainsi, l'invention fournit un procédé de préparation de 1,1,1-trifluoro-2,2-dichloroéthane (F123) par mise en contact de 1,1,1-trifluoro-2-chloroéthane (F133a) avec du chlore, ledit procédé étant caractérisé en ce que la mise en contact est réalisée en présence d'HF et d'un catalyseur dans des conditions de température, de temps de contact et avec des rapports molaires Cl₂/F133a et HF/F133a tels que HF ne réagisse substantiellement pas avec F133a et le F123 formé et favorise la sélectivité en F123, la température étant comprise entre 150 et 300°C, le rapport molaire Cl₂/F133a étant compris entre 0,05 et 0,50 et le rapport molaire HF/F133a étant compris entre 0,5 et 2,5.

L'invention concerne plus particulièrement un procédé tel que HF ne réagisse substantiellement pas avec le F133a et le F123 pour donner des dérivés plus fluorés que le F133a, tels que le F124 ou le F134a.

Dans la mise en oeuvre du procédé selon l'invention, il convient de choisir des conditions opératoires telles que HF se comporte essentiellement comme diluant et/ou stabilisant de la réaction et des réactifs et non pas en tant que réactif dans une réaction de fluoration. D'une manière générale, les conditions de température, les rapports molaires Cl₂/F133a et HF/F133a et le temps de contact peuvent être choisis à l'intérieur des fourchettes connues pour ce genre de réaction de chloration, et telles que rapportées par exemple ci-avant en référence aux documents concernant ladite réaction de chloration.

La température du milieu réactionnel est comprise entre 150 et 300 °C. Cette température est de préférence comprise entre 250 et 300 °C.

Le rapport molaire chlore/F133a est compris entre 0,05 et 0,50 et il est, de préférence, compris entre 0,05 et 0,15.

Le rapport molaire HF/F133a est compris entre 0,5 et 2,5. Pour des raisons pratiques, entre autres liées à la séparation des produits en vue du recyclage des réactifs non consommés, et de l'HF, on choisit de préférence un rapport compris entre 0,8 et 1,2.

Le temps de contact entre F133a, le chlore et HF sur le catalyseur peut être compris entre 5 et 100 secondes ; il est recommandé d'avoir un temps de contact compris entre 10 et 60 secondes.

Les catalyseurs utilisables pour cette invention sont les catalyseurs de chloration connus de l'homme de l'art et résistant aux hydracides tels que HF et HCl. Cependant, on donne la préférence à des catalyseurs généralement utilisés pour les réactions de fluoration par HF. A titre indicatif, on peut citer les catalyseurs massiques ou supportés (sur alumine fluorée ou sur charbon par exemple) à base d'oxyde de Cr, Zn, Ni, Mg seuls ou en mélange. Pour cette invention, on préférera des catalyseurs mixtes composés d'oxydes, d'halogénures et/ou d'oxyhalogénures de nickel et de chrome déposés sur un support constitué de fluorure d'aluminium ou d'un mélange de fluorure d'aluminium et d'alumine tels que décrits par exemple dans les brevets FR 2 669 022 et EP-B-0 609 124.

Préalablement à la réaction de chloration, le catalyseur pourra être conditionné si nécessaire par un traitement thermique en présence de Cl₂ et/ou HF, par exemple en suivant la méthode décrite dans EP-B-0 609 124.

Lorsqu'on utilise un catalyseur mixte nickel/chrome, on recommandera les catalyseurs contenant, en masse, de 0,5 à 20 % de chrome et de 0,5 à 20 % de nickel et plus particulièrement ceux contenant de 2 à 10 % en masse de chacun des métaux dans un rapport atomique nickel/chrome compris entre 0,5 à 5, de préférence voisin de 1.

Ainsi qu'il a été précisé, le procédé conforme à l'invention consiste notamment à effectuer la mise en contact du chlore avec le F133a, en présence d'HF et d'un catalyseur, dans des conditions telles que HF ne réagisse substantiellement pas avec le F133a et le 123 formé pour donner des dérivés plus fluorés. Ces conditions sont avantageusement choisies à l'intérieur des zones de température, rapports molaires, temps de contact indiqués précédemment et il appartiendra à l'homme de l'art de choisir les conditions exactes d'une réaction compte tenu du résultat escompté. Ainsi, à partir du moment où le paramètre de température aura été choisi, par exemple 280 °C, il pourra être avantageux de réduire le rapport molaire Cl₂/F133a, par exemple autour de 10 % pour obtenir la meilleure sélectivité en série 120 au détriment de la série 110. De même, il conviendra, pour une certaine température et un certain taux de chlore, de choisir le temps de contact permettant d'associer le taux de conversion du F133a convenable et une bonne sélectivité en F123. De même encore, le rapport molaire HF/F133a pourra être choisi par exemple en fonction des valeurs désirées ou acceptables pour le rapport molaire série 110/série 120. Ainsi qu'il a été indiqué, ce rapport HF/F133a peut aller généralement de 0,5 à 2,5 compte tenu des autres conditions de réaction (température, temps de contact, rapport molaire Cl₂/F133a), mais, de préférence, ce rapport molaire HF/F133a se situe aux alentours de 1, par exemple entre 0,8 et 1,2.

Les indications qui précèdent montrent à l'évidence que les valeurs recominandées pour les conditions opératoires ont un rôle essentiellement informatif, sachant que l'on ne sortirait pas du cadre de l'invention en retenant, pour l'un ou l'autre des paramètres de la réaction, des valeurs situées au-delà ou en deçà des valeurs indiquées plus haut, pour autant que de telles modifications opératoires n'impliquent pas une réaction de HF avec le F133a conduisant à la formation de quantités substantielles de dérivés plus fluorés.

La réaction de chloration peut être conduite en phase gazeuse, en lit fixe ou en lit fluide, en batch, ou, de préférence, en continu, avec possibilité de recyclage dans le réacteur des réactifs non transformés et de l'HF. L'acide chlorhydrique formé lors de la réaction est séparé de préférence avant le recyclage. Le F123 récupéré peut être purifié par distillation selon la pureté désirée.

Le chlore peut être introduit dans le réacteur pur ou dilué dans un gaz inerte tel que l'azote. Les matériaux utilisés pour la construction de l'installation doivent être compatibles avec la présence de chlore et d'hydracides tels que HCl et HF ; ils peuvent être choisis par exemple en "Hastelloy" ou en "Inconel" qui sont résistants aux milieux corrosifs contenant ces hydracides.

La réaction de chloration selon l'invention peut être menée à la pression atmosphérique ou à une pression supérieure à cette dernière. Pour des raisons pratiques, on opère généralement dans une zone allant de 0 à 25 bars relatifs et de préférence entre 0 et 15 bars relatifs.

Dans des conditions opératoires susceptibles d'encrasser le catalyseur, il peut être judicieux d'introduire avec les réactifs, de l'oxygène à faible teneur. Cette teneur peut varier selon les conditions opératoires entre 0,02 et 5 % par rapport aux réactifs organiques (pourcentage molaire). L'oxygène pourra être introduit de manière continue ou séquentielle.

Le procédé conforme à l'invention permet de préparer du F123 à partir du F133a, dans des conditions de température et de pression modérées, et avec une excellente sélectivité en F123.

Le 1,1,1-trifluoro-2-chloroéthane (F133a) de départ peut lui-même être obtenu par application de procédés maintenant bien connus. Le F133a peut notamment être préparé par fluoration du trichloroéthylène (par exemple en suivant la méthode préconisée par Mc Bee et al., Ind. Eng. Chem. 39, 409-412), par fluoration du F132b, par fluoration du F130a, par hydrogénolyse du F113a.

Dans l'invention, on donne la préférence au F133a obtenu par fluoration du trichloroéthylène.

A ce titre, l'invention a également pour objet un procédé de préparation de F123, à partir de trichloroéthylène, ledit procédé comprenant :
a) une étape de fluoration du trichloroéthylène, dans une réaction, en phase liquide ou phase gazeuse, en présence d'un catalyseur et sous une pression conduisant après séparation d'HCl et des lourds, à un mélange de F133a accompagné d'HF, entraîné sous forme azéotropique ;
b) une étape de chloration du F133a, par mise en contact dudit F133a avec du chlore, en présence de HF et d'un catalyseur, dans des conditions de température, de temps de contact et avec des rapports Cl₂/F133a et HF/F133a tels que HF ne réagisse substantiellement pas avec le F133a et le F123 formé.

Dans la phase a), pour un procédé en phase liquide, on utilise de préférence un catalyseur à base de sels d'antimoine et on opère avantageusement sous une pression d'au moins 10 bars absolus. On peut également faire réagir le trichloroéthylène avec HF en présence d'oxyde de chrome ou d'oxyfluorure de chrome dans un procédé en phase gazeuse.

Le F123 peut être utilisé tel quel, par exemple comme agent propulseur en réfrigération et dans la fabrication de mousses, où il remplace avantageusement le F11 du fait de son innocuité sur l'ozone de la stratosphère. De ce fait, le procédé conforme à l'invention qui, par les résultats auxquels il conduit, est exploitable au niveau industriel, est particulièrement intéressant, tant au départ de trichloroéthylène qu'à celui du F133a.

Le F123 peut aussi subir une fluoration supplémentaire et conduire ainsi au F125 (pentafluoroéthane). Cette réaction peut être conduite selon divers procédés maintenant connus : d'une manière générale, cette étape comprend la mise en contact du F123 (seul ou en mélange avec d'autres composés de la série 120) avec de l'HF en présence d'un catalyseur de fluoration pour obtenir du F125.

Cette étape peut être réalisée en phase liquide ou en phase vapeur, et le catalyseur peut être choisi parmi les catalyseurs dont l'emploi est décrit par exemple, dans EP-B-609 124 ou dans les références auxquelles ce brevet renvoie, ledit brevet étant incorporé ici notamment pour les conditions recommandées pour cette réaction.

L'invention a donc encore pour objet un procédé de préparation de pentafluoroéthane (F125), ledit procédé comprenant :
a) une étape de fluoration de trichloroéthylène telle que décrite précédemment et aboutissant notamment à du F133a ;
b) une étape de chloration du F133a, telle que décrite précédemment et aboutissant notamment à du F123 ;
c) une étape de fluoration de F123, par mise en contact de F123 avec HF, en présence d'un catalyseur, avec ou sans recyclage de F124, la fluoration du F124 pouvant faire l'objet d'une étape séparée.

Dans cette étape, le catalyseur est, avantageusement, un catalyseur mixte composé d'oxydes, d'halogénures et/ou d' oxyhalogénures de nickel et de chrome tel que décrit dans EP-B-609 124. On peut également utiliser les catalyseurs à base d'oxyde ou d'oxyfluorure de chrome ou à base d'alumine ou de fluorure d'aluminium, éventuellement dopé par un métal tel que zinc, nickel ou fer. De tels catalyseurs sont décrits par exemple dans EP-502 605 ou WO 93/16 798. On peut encore utiliser un catalyseur du type chrome/charbon tel que décrit par exemple dans EP-A-456 552.

Pour cette étape, on utilise de préférence un catalyseur mixte décrit ci-dessus déposé sur un support constitué de fluorure d'aluminium ou d'un mélange de fluorure d'aluminium et d'alumine.

La température de cette réaction de fluoration peut être comprise entre 250 et 470 °C et est de préférence comprise entre 280 et 410 °C. Le temps de contact entre HF et F123 peut être compris entre 3 et 100 s, de préférence entre 5 et 30 s. Le rapport molaire HF/F123 peut aller de 1/1 à 20/1 et de préférence de 2/1 à 9/1.

Bien que la réaction puisse être conduite à pression atmosphérique, on préfère opérer sous légère pression, par exemple n'excédant pas 10 bars absolus et même inférieure à 5 bars absolus.

Le F125 obtenu peut être ensuite purifié par exemple par application des méthodes décrites dans FR-2 758 137 ou WO 95/21147 dont le contenu est incorporé ici par référence.

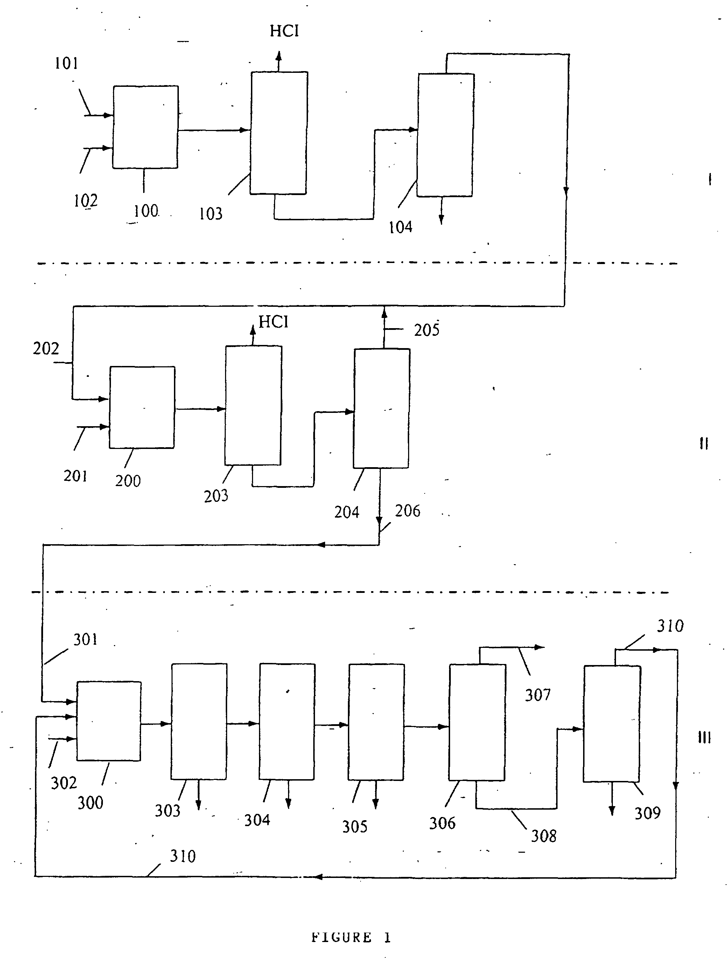

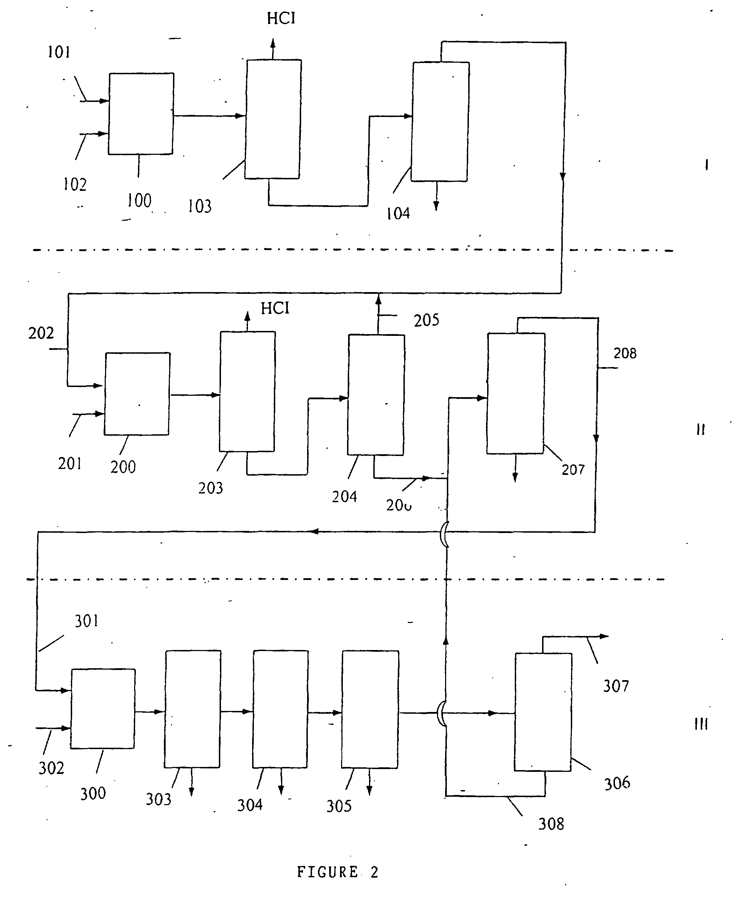

Le procédé de préparation de F125 à partir de trichloroéthylène, qui constitue également un objet de l'invention, peut être mis en oeuvre en continu dans une installation telle que présentée schématiquement par la figure unique.

Cette figure constitue un schéma d'ensemble des trois étapes I, II et III du procédé en question.

L'installation comprend notamment :
Etape I
   - un réacteur (100), contenant le catalyseur ;
   - des arrivées de trichloroéthylène (101) et d'HF (102) ;
   - une colonne de distillation d'HCl (103) ;
   - une colonne de séparation de F133a + HF des lourds (104) :
Etape II
   - un réacteur de chloration (200) alimenté par
   - du F133a et de l'HF (202) ;
   - du chlore (201) ;
   - une colonne de séparation d'HCl (203) ;
   - une colonne (204) de séparation du F123 brut du F133a n'ayant pas réagi (+ notamment HF azéotropique + Cl₂ n'ayant pas réagi) qui sont recyclés ;
Etape III
   - un réacteur de fluoration (300), alimenté en F123 + F123a (207/301), en HF (302) et éventuellement en F124 ;
   - une colonne (306) permettant de séparer le F125 (307), le pied de colonne étant recyclé (308) dans la colonne (206) de l'étape II ;
   - à la sortie du réacteur (300), les gaz réactionnels subissent les lavages habituels à l'acide sulfurique (303), à l'eau (304) et à la soude (305) pour récupérer HF, HCl et neutraliser le milieu avant distillation (306).

Bien entendu, l'installation industrielle comprend des dispositifs complémentaires dont l'usage est connu (purge, évaporateurs, surchauffeur, décanteurs).

L'invention concerne donc également une installation de production de F125 à partir de trichloroéthylène, et qui comprend au moins la succession de dispositifs tels que représentés sur la figure.

L'invention sera maintenant illustrée par les exemples suivants, qui ne sont donnés qu'à titre purement indicatif.

### Exemple 1

Dans un tube en Inconel de diamètre interne 21 mm, on introduit 75 cm³ de catalyseur Ni-Cr/AlF₃ (préparé comme décrit dans le brevet FR 2 669 022). Le catalyseur est traité pendant 15 h avec 1 mole/h d'HF anhydre à 350 °C et sous pression atmosphérique. Préalablement à la réaction, le débit d'HF est ajusté à 1,13 mole/h et la température à 280 °C. Ensuite, un mélange Cl₂/N₂ à 15 % molaire de chlore est introduit dans le réacteur avec un débit de 0,79 mole/h. Enfin, CF₃-CH₂CI est introduit dans le réacteur avec un débit de 1,02 mole/h et la pression totale de réaction est régulée à 15 bars. Après 6 h de réaction, un échantillon gazeux est prélevé pour analyse en chromatographie en phase gazeuse. Avant le prélèvement, le gaz est débarrassé de l'HF, de l'HCI et du chlore par bullage dans des flacons laveurs à eau et soude/sulfite puis séché sur CaCl₂.

La conversion de F133a est de 7 % pour une sélectivité en F123 de 93 %. Le ratio série 110/série 120 est de 3,4%.

### Exemples 2 à 7

Suivant le même protocole que dans l'exemplel, différentes conditions ont été testées :

| Conditions | Ex.2 | Ex.3 | Ex.4 | Ex.5 | Ex.6 | Ex.7 |
|---|---|---|---|---|---|---|
| Température (°C) | 280 | 280 | 280 | 310 | 280 | 280 |
| RMCl₂/F133a | 0,13 | 0,47 | 0,13 | 0,14 | 0,1 | 0,1 |
| RM HF/F133a | 2 | 2,3 | 2,1 | 2,1 | 1 | 0,7 |
| tc(s) | 20 | 13 | 38 | 19 | 15 | 32 |
| Résultats | | | | | | |
| Conversion F133a % | 6,2 | 13,3 | 10,1 | 12,2 | 4,3 | 10 |
| Sélectivité F123 % | 93 | 87 | 93 | 85 | 93 | 74 |
| Ratio série 110/120 | 2,8% | 7,8% | 4% | 5% | 1,8% | 6,4% |

### Exemples comparatifs :

### Exemple 1a (sans HF)

Dans un tube en Inconel de diamètre interne 21 mm, on introduit 75 cm³ de catalyseur Ni-Cr/AlF₃ (préparé comme décrit dans le brevet FR 2 669 022). Le catalyseur est traité pendant 15 h avec 1 mole/h d'HF anhydre à 350 °C et pression atmosphérique. Préalablement à la réaction, le débit d'HF est arrêté et la température réduite à 280 °C. Ensuite, un mélange Cl₂/N₂ à 15 % molaire de chlore est introduit dans le réacteur avec un débit de 1,13 mole/h. Enfin, CF₃-CH₂Cl est introduit dans le réacteur avec un débit de 1,63 mole/h et la pression totale de réaction est régulée à 15 bars. Après 6 h de réaction, un échantillon gazeux est prélevé pour analyse en chromatographie en phase gaz. Avant le prélèvement, le gaz est débarrassé de l'HF, de l'HCl et du chlore par bullage dans des flacons laveurs à eau et soude/sulfite puis séché sur CaCl₂.

La conversion de F133a est de 10 %, pour une sélectivité de F123 de 42 %. Le ratio série 110/série 120 est de 37%.

### Exemple 1 b : sans HF et sans catalyseur

Dans un tube en Inconel vide de diamètre interne 21 mm, on introduit un mélange Cl₂/N₂ à 15 % molaire de chlore avec un débit de 1,3 mole/h. Ensuite, CF₃-CH₂Cl est introduit dans le réacteur avec un débit de 2,08 moles/h et la pression totale de réaction est régulée à 15 bars.

Après 6 h de réaction, un échantillon gazeux est prélevé pour analyse en chromatographie en phase gaz. Avant le prélèvement, le gaz est débarrassé de l'HF, de l'HCl et du chlore par bullage dans des flacons laveurs à eau et soude/sulfite puis séché sur CaCl₂.

La conversion de F133a est de 2 % pour une sélectivité en F123 de 79 %. Le ration série 110/série 120 est de 15,4%.

## Revendications

1. Procédé de préparation de 1,1,1-trifluoro-2,2-dichloroéthane (F123) par mise en contact de 1,1,1-trifluoro-2-chloroéthane (F133a) avec du chlore, ledit procédé étant **caractérisé en ce que** la mise en contact est réalisée en présence d'HF et d'un catalyseur dans des conditions de température, de temps de contact et avec des rapports molaires Cl₂/F133a et HF/F133a tels que HF ne réagisse substantiellement pas avec F133a et le F123 formé et favorise la sélectivité en F123, la température étant comprise entre 150 et 300°C, le rapport molaire Cl₂/F133a étant compris entre 0,05 et 0,50 et le rapport molaire HF/F133a étant compris entre 0,5 et 2,5.

2. Procédé selon la revendication 1, dans lequel la température est comprise entre 250 et 300°C.

3. Procédé selon la revendication 1 ou 2, dans lequel le rapport molaire Cl₂/F133a est compris entre 0,05 et 0,15.

4. Procédé selon l'une quelconque des revendications 1 à 3, dans lequel le rapport molaire HF/F133a est compris entre 0,8 et 1,2.

5. Procédé selon l'une quelconque des revendications 1 à 4, dans lequel le temps de contact entre F133a, Cl₂, et d'HF sur le catalyseur est compris entre 5 et 100 secondes.

6. Procédé selon la revendication 5, dans lequel le temps de contact est compris entre 10 et 60 secondes.

7. Procédé selon l'une quelconque des revendications 1 à 6, dans lequel le catalyseur est choisi parmi les catalyseurs mixtes composé d'oxydes, d'halogénures et/ou d'oxyhalogénures de nickel et de chrome.

8. Procédé selon la revendication 7, dans lequel le catalyseur est déposé sur un support constitué de fluorure d'aluminium ou d'un mélange de fluorure d'aluminium et d'alumine.

9. Procédé selon l'une quelconque des revendications 1 à 8, **caractérisé en ce qu**'il est mis en oeuvre en continu.

10. Procédé selon l'une quelconque des revendications 1 à 9, dans lequel le F133a de départ est obtenu par fluoration du trichloroéthylène.

11. Procédé selon la revendication 10, dans lequel la fluoration du trichloroéthylène est réalisée en phase liquide sous pression, en présence d'un cataly seur à base de sels d'antimoine ou en phase gazeuse en présence d'un catalyseur à base d'oxyde de chrome ou d'oxyfluorure de chrome.

12. Procédé selon l'une quelconque des revendications 1 à 11, suivi d'une étape additionnelle de fluoration du F123 pour la préparation de pentafluoroéthane (F125).

13. Procédé selon la revendication 12, dans lequel on met le F123 en contact avec HF en présence d'un catalyseur tel que décrit dans la revendication 7.

## Patentansprüche

1. Verfahren zur Herstellung von 1,1,1-Trifluor-2,2-dichlorethan (F123) durch In-Kontakt-bringen von 1,1,1-Trifluor-2-chlorethan (F133a) mit Chlor, wobei das Verfahren **dadurch gekennzeichnet ist, daß** das In-Kontakt-bringen in Gegenwart von HF und eines Katalysators unter solchen Temperatur- und Kontaktzeitbedingungen und bei Molverhältnissen Cl_{2/}F133a und HF/F133a durchgeführt wird, daß HF im wesentlichen nicht mit F133a und dem gebildeten F123 reagiert und die Selektivität für F123 begünstigt wird, wobei die Temperatur zwischen 150 und 300°C liegt, das Molverhältnis Cl₂/F133a zwischen 0,05 und 0,50 liegt und das Molverhältnis HF/F133a zwischen 0,5 und 2,5 liegt.

2. Verfahren gemäß Anspruch 1, bei dem die Temperatur zwischen 250 und 300°C liegt.

3. Verfahren gemäß Anspruch 1 oder 2, bei dem das Molverhältnis Cl_{2/}F133a zwischen 0,05 und 0,15 liegt.

4. Verfahren gemäß einem der Ansprüche 1 bis 3, bei dem das Molverhältnis HF/F133a zwischen 0,8 und 1,2 liegt.

5. Verfahren gemäß einem der Ansprüche 1 bis 4, bei dem die Kontaktzeit zwischen F133a, Cl₂ und HF auf dem Katalysator zwischen 5 und 100 Sekunden liegt.

6. Verfahren gemäß Anspruch 5, bei dem die Kontaktzeit zwischen 10 und 60 Sekunden liegt.

7. Verfahren gemäß einem der Ansprüche 1 bis 6, bei dem der Katalysator aus den aus Oxiden, Halogeniden und/oder Oxyhalogeniden von Nickel und Chrom bestehenden Mischkatalysatoren ausgewählt ist.

8. Verfahren gemäß Anspruch 7, bei dem der Katalysator auf einem aus Aluminiumfluorid oder einem Gemisch von Aluminiumfluorid und Aluminiumoxid bestehenden Träger niedergeschlagen ist.

9. Verfahren gemäß einem der Ansprüche 1 bis 8, **dadurch gekennzeichnet, daß** es kontinuierlich durchgeführt wird.

10. Verfahren gemäß einem der Ansprüche 1 bis 9, bei dem das eingesetzte F133a durch Fluorierung von Trichlorethylen erhalten wurde.

11. Verfahren gemäß Anspruch 10, bei dem die Fluorierung des Trichlorethylens in flüssiger Phase unter Druck in Gegenwart eines Katalysators auf der Grundlage von Antimonsalzen oder in der Gasphase in Gegenwart eines Katalysators auf der Grundlage von Chromoxid oder Chromoxyfluorid durchgeführt wird.

12. Verfahren gemäß einem der Ansprüche 1 bis 11, das von einem weiteren Schritt der Fluorierung von F123 zur Herstellung von Pentafluorethan (F125) gefolgt wird.

13. Verfahren gemäß Anspruch 12, bei dem man das F123 mit HF in Gegenwart eines wie in Anspruch 7 beschriebenen Katalysators in Kontakt bringt.

## Claims

1. Process for the preparation of 1,1,1-trifluoro-2,2-dichloroethane (F123) by bringing 1,1,1-trifluoro-2-chloroethane (F133a) into contact with chlorine, the said process being **characterized in that** the contacting operation is carried out in the presence of HF and of a catalyst under conditions of temperature and of contact time and with Cl_{2/}F133a and HF/F133a molar ratios such that HF does not substantially react with the F133a and the F123 formed and promotes the selectivity for F123, in which the temperature is between 150 and 300°C, and in which the HF/F133a molar ratio is between 0.5 and 2.5.

2. Process according to Claim 1, in which the temperature is between 250 and 300°C.

3. Process according to Claim 1 or 2, in which the Cl_{2/}F133a molar ratio is between 0.05 and 0.15.

4. Process according to any one of Claims 1 to 3, in which the HF/F133a molar ratio is between 0.8 and 1.2.

5. Process according to any one of Claims 1 to 4, in which the time for contact between F133a, Cl₂ and HF over the catalyst is between 5 and 100 seconds.

6. Process according to Claim 5, in which the contact time is between 10 and 60 seconds.

7. Process according to any one of Claims 1 to 6, in which the catalyst is chosen from mixed catalysts composed of nickel and chromium oxides, halides and/or oxyhalides.

8. Process according to Claim 7, in which the catalyst is deposited on a support composed of aluminium fluoride or of a mixture of aluminium fluoride and of alumina.

9. Process according to any one of Claims 1 to 8, **characterized in that** it is carried out continuously.

10. Process according to any one of Claims 1 to 9, in which the starting F133a is obtained by fluorination of trichloroethylene.

11. Process according to Claim 10, in which the fluorination of the trichloroethylene is carried out in the liquid phase under pressure in the presence of a catalyst based on antimony salts or in the gas phase in the presence of a catalyst based on chromium oxide or on chromium oxyfluoride.

12. Process according to any one of Claims 1 to 11 followed by an additional step of fluorination of the F123 for the preparation of pentafluoroethane (F125).

13. Process according to Claim 12, in which the F123 is brought into contact with HF in the presence of a catalyst as described in Claim 7.
